# EUROPÄISCHE PATENTANMELDUNG

(11) **EP 3 610 902 A1**
(43) Veröffentlichungstag der Anmeldung: **19.02.2020**
(21) Anmeldenummer: 18189536.8
(22) Anmeldetag: 17.08.2018
(51) Int. Cl.: A61M 1/10

(54) **PUMPENSYSTEM ZUM PUMPEN EINES FLUIDS UND VERFAHREN ZUM BETRIEB DES PUMPENSYSTEMS**

(71) Anmelder: Berlin Heart GmbH, 12247 Berlin (DE)
(72) Erfinder: Steingräber, Dr.-Ing. Robert, 14055 Berlin (DE)
(74) Vertreter: Pfenning, Meinig & Partner mbB

(57) **Zusammenfassung**

Die vorliegende Erfindung betrifft ein Pumpensystem zum Pumpen eines Fluids sowie ein Verfahren zum Betrieb des Pumpensystems. Das Pumpensystem umfasst eine erste Membranfluidpumpe, eine mit der ersten Membranfluidpumpe verbundene erste Einlasskanüle zum Zuleiten eines Fluids zur ersten Membranfluidpumpe sowie eine mit der ersten Membranfluidpumpe verbundene erste Auslasskanüle zum Ableiten des Fluids aus der ersten Membranfluidpumpe, eine erste Arbeitspumpe, welche über eine erste Druckleitung mit der ersten Membranfluidpumpe verbunden ist und eingerichtet ist, über die erste Druckleitung die erste Membranfluidpumpe anzutreiben. Das Pumpensystem umfasst weiterhin einen ersten Einlassflusssensor zum Erfassen eines ersten Einlassflusses des Fluids in der ersten Einlasskanüle und/oder einen ersten Auslassflusssensor zum Erfassen eines ersten Auslassflusses des Fluids in der ersten Auslasskanüle.

## Beschreibung

Die vorliegende Erfindung betrifft ein Pumpensystem zum Pumpen eines Fluids sowie ein Verfahren zum Betrieb des Pumpensystems. Die Erfindung betrifft weiterhin ein Herzunterstützungssystem zur Unterstützung eines menschlichen oder tierischen Herzens.

Im Stand derTechnik sind Pumpensysteme zum Pumpen von Blut als Teil von Herzunterstützungssystemen bekannt. Derartige Pumpensysteme können zur ein- oder zweiseitigen Herzunterstützung ausgebildet sein. Das Pumpensystem umfasst mindestens eine Membranfluidpumpe, die mit einer Einlasskanüle zum Führen von Blut von einer Herzkammer oder einem Vorhof eines Herzens zur Membranfluidpumpe sowie mit einer Auslasskanüle zum Führen von Blut von der Membranfluidpumpe zu einem Blutgefäß verbunden ist. Weiterhin umfasst das Pumpensystem mindestens eine Arbeitspumpe, welche über eine Druckleitung mit der Membranfluidpumpe verbunden ist und über die Druckleitung die Membranfluidpumpe antreibt.

Als Antrieb für die Membranfluidpumpe sind verschiedene Pneumatikantriebe bekannt. Eine Antriebsvariante sieht eine Kolbenpumpe als Arbeitspumpe vor, welche einen Pneumatikzylinder mit einem darin axial beweglichen Arbeitskolben umfasst. Der Arbeitskolben kann dabei derart bewegt werden, dass ein Arbeitsraum, der in einer Druckaustauschverbindung mit der Druckleitung steht, abwechselnd verkleinert und vergrößert wird. Der Pneumatikzylinder weist weiterhin ein Ausgleichsventil auf, über das Luft aus der Umgebung in den Arbeitsraum einströmen oder aus dem Arbeitsraum in die Umgebung ausströmen kann. Außerdem verfügt das Pumpensystem über mindestens eine Steuereinheit zum Steuern der Bewegung des Arbeitskolbens sowie zum Steuern der in dem Arbeitsraum enthaltenen Luftmasse.

Der Arbeitskolben wird durch einen Elektromotor angetrieben. Dabei steuert die Steuereinheit den Elektromotor so, dass sich der Arbeitskolben axial innerhalb des Pneumatikzylinders hin und her bewegt. Bewegt sich der Arbeitszylinder zur Druckleitung hin, wird der Arbeitsraum verkleinert und der Druck im Arbeitsraum und in der Druckleitung erhöht. Durch den höheren Druck wölbt sich die Membran der Membranfluidpumpe zur Auslasskanüle hin vor und drückt das Blut aus der Membranfluidpumpe durch die Auslasskanüle in das mit der Auslasskanüle verbundene Blutgefäß. Anschließend kehrt sich die Bewegung des Arbeitszylinders um, so dass der Druck im Arbeitsraum und der Druckleitung abnimmt. Dadurch wölbt sich nun die Membran der Membranfluidpumpe zur Druckleitung hin vor und saugt das Blut durch die Einlasskanüle aus der Herzkammer oder dem Vorhof in die Membranfluidpumpe.

Eine zweite Antriebsvariante umfasst einen Unterdruckbehälter, einen Kompressor, einen Überdruckbehälter und ein Ventil, welche der Reihe nach in einem Pneumatikschaltkreis miteinander verbunden sind, als Arbeitspumpe. Das Ventil ist dabei mit der Druckleitung verbunden, sodass über das Ventil abwechselnd ein im Unterdruckbehälter anliegender Unterdruck und ein im Überdruckbehälter anliegender Überdruck auf die Druckleitung übertragen werden. Über die Druckleitung wird der abwechselnd anliegende Unter- und Überdruck auf die Membran der Membranfluidpumpe übertragen und so das Blut abwechselnd in die Membranfluidpumpe gesaugt und aus der Membranfluidpumpe herausgedrückt.

Bei Laständerungen besteht die Gefahr, dass die Blutkammer der Membranfluidpumpe nicht mehr vollständig gefüllt oder entleert wird, wodurch sich der mittlere Blutfluss verringert. Dabei ist unter vollständigem Füllen und Entleeren eine vollständige Bewegung der Membran in ihre Endlagen gemeint. Am Ende der Entleerungsphase kann ein Restblutvolumen, am Ende der Füllungsphase auf der Luftseite ein Restluftvolumen vorhanden sein. Mögliche Folgen des verringerten Blutflusses können die Bildung von Thromben oder eine Unterversorgung des Patienten sein. Theoretisch könnten Laständerungen durch hohe Druckreserven ausgeglichen werden. Hohe Drücke im Pumpensystem führen jedoch einerseits zu einem sehr kurzen Fluss und können das Blut schädigen. Eine Verringerung des Blutflusses sowie hohe Drücke sollen somit vermieden werden, um eine Unterversorgung zu verhindern, die Belastung des Bluts zu reduzieren und keine Stagnationszeiten entstehen zu lassen. Insbesondere bei einem mobilen Einsatz des Pumpensystems können derartige Laständerungen vermehrt auftreten. Die Aufgabe der vorliegenden Erfindung ist es daher, ein Pumpensystem sowie ein Herzunterstützungssystem enthaltend ein derartiges Pumpensystembereitzustellen, welches eine Überwachung eines Flusses in der Einlass- und/oder der Auslasskanüle sowie eines Füllungs- und Entleerungsgrads der Membranfluidpumpe in jedem Pumpzyklus ermöglicht. Weiterhin ist es die Aufgabe der vorliegenden Erfindung ein Verfahren zum Betrieb eines derartigen Pumpensystems bereitzustellen.

Das erfindungsgemäße Pumpensystem zum Pumpen eines Fluids umfasst eine erste Membranfluidpumpe, eine mit der ersten Membranfluidpumpe verbundene erste Einlasskanüle zum Zuleiten eines Fluids zur ersten Membranfluidpumpe sowie eine mit der ersten Membranfluidpumpe verbundene erste Auslasskanüle zum Ableiten des Fluids aus der ersten Membranfluidpumpe, und eine erste Arbeitspumpe, welche über eine erste Druckleitung mit der ersten Membranfluidpumpe verbunden ist und eingerichtet ist, über die erste Druckleitung die erste Membranfluidpumpe anzutreiben. Das erfindungsgemäße Pumpensystem zeichnet sich durch einen ersten Einlassflusssensor zum Erfassen eines ersten Einlassflusses des Fluids in der ersten Einlasskanüle und/oder einen ersten Auslassflusssensor zum Erfassen eines ersten Auslassflusses des Fluids in der ersten Auslasskanüle aus. Durch den Einlassflusssensor bzw. den Auslassflusssensor ist es mit dem erfindungsgemäßen Pumpensystem möglich eine Änderung des Einlassflusses bzw. des Auslassflusses, insbesondere einen Rückgang des Einlass- bzw. Auslassflusses, unmittelbar zu erkennen und das System ggf. so nachzuregeln, dass die Änderung des Einlass- bzw. Auslassflusses kompensiert wird. Derartige Einlass- bzw. Auslassflussänderungen können insbesondere die Folge von Laständerungen, insbesondere bei einem mobilen Einsatz des Pumpensystems, oder bei Veränderungen in dem Pumpensystem sein. Mittels des Einlass- bzw. Auslassflusssensors können so auch Laständerungen erkannt und ggf. kompensiert werden. Hierdurch wird wiederum die Gefahr der Thrombenbildung und der Unterversorgung des Patienten reduziert.

Im Folgenden sollen zunächst einige in der vorliegenden Beschreibung der Erfindung verwendete Begriffe erklärt werden. Unter dem Begriff Entleerungsphase wird hier die Phase der Ausströmung des Fluids aus der Membranfluidpumpe verstanden. Unter dem Begriff Füllungsphase wird dagegen die Phase der Einströmung des Fluids in die Membranfluidpumpe verstanden. Unter den Begriffen Entleerungs- und Füllungsdauer wird entsprechend die Dauer der jeweiligen Phase verstanden. Unter dem Begriff relative Entleerungsdauer, wird die Dauer der Entleerungsphase relativ zur Dauer der Füllungsphase verstanden. Unter dem Begriff Druck wird der momentane und relativ zum Umgebungsdruck gemessene Druck verstanden. Unter dem Begriff Mitteldruck wird der relativ zum Umgebungsdruck gemessene und über einen Pumpzyklus gemittelte Druck im Arbeitsraum und in der Druckleitung verstanden. Ein Pumpzyklus setzt sich aus einer Entleerungsphase und einer Füllungsphase zusammen. Dementsprechend stellen die Entleerungsphase und die Füllungsphase jeweils einen halben Pumpzyklus dar.

Das erfindungsgemäße Pumpensystem eignet sich insbesondere, jedoch nicht ausschließlich, zum Pumpen von Blut. Das erfindungsgemäße Pumpensystem kann insbesondere Teil eines Herzunterstützungssystems sein. Jedoch sind auch andere Anwendungen des erfindungsgemäßen Pumpensystems denkbar.

In einer bevorzugten Weiterbildung des erfindungsgemäßen Pumpensystems kann das Pumpensystem eine Steuereinheit zum Steuern der ersten Arbeitspumpe basierend auf dem erfassten ersten Einlass- und/oder ersten Auslassfluss, insbesondere um eine Vorgabe für einen erfassten ersten Einlass- und/oder Auslassfluss zu erreichen, umfassen. Unter Steuern der ersten Arbeitspumpe basierend auf dem erfassten ersten Einlass- und/oder ersten Auslassfluss kann hier insbesondere eine gezielte Beeinflussung von Stellgliedern der ersten Arbeitspumpe verstanden werden, sodass ein vorgegebener erster Einlass- und/oder erster Auslassfluss zumindest näherungsweise erreicht wird. Insbesondere kann unter Steuern der ersten Arbeitspumpe basierend auf dem erfassten ersten Einlass- und/oder ersten Auslassfluss eine Regelung zum Erreichen des vorgegebenen ersten Einlass- und/oder ersten Auslassflusses verstanden werden.

Die Steuereinheit kann auch eingerichtet sein, basierend auf dem erfassten ersten Einlass- und/oder Auslassfluss eine Vorgabe für den ersten Einlass- und/oder Auslassfluss zu bestimmen und die Arbeitspumpe gemäß dieser Vorgabe zu steuern. Die Vorgabe für den ersten Einlass- und/oder Auslassfluss kann eine Vorgabe für einen zeitlichen Mittelwert oder ein Zeitverhalten des ersten Einlass- und/oder Auslassflusses umfassen.

Weiterhin kann die Steuereinheit eingerichtet sein, basierend auf dem erfassten ersten Einlass- und/oder ersten Auslassfluss oder basierend auf der Vorgabe für den ersten Einlass- und/oder Auslassfluss eine Vorgabe für einen in der ersten Druckleitung anliegenden Mitteldruck, eine Vorgabe für eine in der ersten Druckleitung anliegende Druckamplitude, eine Vorgabe für eine Pumprate der ersten Arbeitspumpe, eine Vorgabe für eine relative Entleerungsdauer, eine Vorgabe für einen in der ersten Druckleitung anliegenden Entleerungsdruck und/oder eine Vorgabe für einen in der ersten Druckleitung anliegenden Füllungsdruck zu bestimmen, und die erste Arbeitspumpe gemäß der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck so zu steuern, dass ein vorgegebener erster Einlass- und/oder Auslassfluss im Wesentlichen erreicht wird.

Sämtliche vorstehend und nachfolgend genannten Drücke innerhalb des Pumpensystems können insbesondere Relativdrücke, also relativ zum Umgebungsluftdruck gemessene Drücke, sein. Unter Steuern der Arbeitspumpe gemäß den vorstehend genannten Vorgaben wird hier insbesondere eine Regelung zum Erreichen des vorgegebenen ersten Einlass- und/oder ersten Auslassflusses verstanden, wobei der erste Einlass- und/oder erste Auslassfluss Regelgrößen darstellen können und entsprechend der Mitteldruck, die Druckamplitude, die Pumprate, die relative Entleerungsdauer, der Entleerungsdruck und/oder der Füllungsdruck Stellgrößen darstellen können.

In einer vorteilhaften Ausgestaltung der vorliegenden Erfindung kann die Arbeitspumpe als Kolbenpumpe ausgestaltet sein. Die Arbeitspumpe kann insbesondere einen in einem Arbeitsraum oszillatorisch bewegbaren Arbeitskolben und ein Ausgleichsventil zum Ändern einer Masse eines Arbeitsfluids im Arbeitsraum und/oder eines Drucks im Arbeitsraum umfassen, wobei sich der Arbeitsraum in einer Druckaustauschverbindung mit der ersten Druckleitung befindet, und die Steuereinrichtung eingerichtet ist, basierend auf dem erfassten ersten Einlass- und/oder ersten Auslassfluss und/oder basierend auf der Vorgabe für den ersten Einlass- und/oder Auslassfluss eine Vorgabe für einen Mitteldruck im Arbeitsraum, eine Vorgabe für eine Druckamplitude im Arbeitsraum, eine Vorgabe für eine Pumprate, eine Vorgabe für einen Hub des Arbeitskolbens, eine Vorgabe für eine Kraft des Arbeitskolbens, eine Vorgabe für eine relative Entleerungsdauer, eine Vorgabe für einen Entleerungsdruck und/oder eine Vorgabe für einen Füllungsdruck im Arbeitsraum zu bestimmen, und die Bewegung des Arbeitskolbens und das Ausgleichsventil gemäß der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck so zu steuern, dass ein vorgegebener erster Einlass- und/oder erste Auslassfluss im Wesentlichen erreicht wird. Unter Steuern der Bewegung des Arbeitskolbens und des Ausgleichsventils gemäß den vorstehend genannten Vorgaben wird hier insbesondere eine Regelung zum Erreichen des vorgegebenen ersten Einlass- und/oder ersten Auslassflusses verstanden, wobei der erste Einlass- und/oder erste Auslassfluss Regelgrößen darstellen können und entsprechend der Mitteldruck, die Druckamplitude, die Pumprate, der Hub, die Kraft, die relative Entleerungsdauer, der Entleerungsdruck und/oder der Füllungsdruck Stellgrößen darstellen können.

Die Kraft des Arbeitskolbens kann insbesondere eine Last und eine Trägheit des Arbeitskolbens einschließen.

Weiterhin kann die Steuereinheit eingerichtet sein, basierend auf der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck eine Positionsvorgabe für den Arbeitskolben zu bestimmen und die Bewegung des Arbeitskolbens gemäß der Positionsvorgabe und das Ausgleichsventil gemäß der Vorgabe für den Mitteldruck so zu steuern, dass die Vorgabe für den Mitteldruck, die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck im Wesentlichen erreicht wird. Unter Steuern der Bewegung des Arbeitskolbens und des Ausgleichsventils gemäß den vorstehend genannten Vorgaben wird hier insbesondere eine Regelung zum Erreichen der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck verstanden, wobei diese Vorgaben in Vorgaben für einen Druck- und Positionsregler umgerechnet werden.

Die Positionsvorgabe kann eine Vorgabe für das Zeitverhalten der Position oder eine Vorgabe für einen oder beide Umkehrpunkte des Arbeitskolbens umfassen.

Weiterhin kann die Steuereinheit eingerichtet sein, basierend auf der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck eine Druckvorgabe für den Arbeitsraum und eine Vorgabe für eine Umkehrposition des Arbeitskolbens zu bestimmen und die Bewegung des Arbeitskolbens gemäß der Druckvorgabe und das Ausgleichsventil gemäß der Vorgabe für die Umkehrposition so zu steuern, dass die Vorgabe für den Mitteldruck, die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck im Wesentlichen erreicht wird. Unter Steuern der Bewegung des Arbeitskolbens und des Ausgleichsventils gemäß den vorstehend genannten Vorgaben wird hier insbesondere eine Regelung zum Erreichen der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck verstanden, wobei diese Vorgaben in eine Vorgabe für einen Druckregler umgerechnet werden.

Die Druckvorgabe für den Arbeitsraum kann eine Vorgabe für ein Zeitverhalten des Drucks, eine Vorgabe für einen zeitabhängigen Druckwert oder eine Vorgabe für einen positionsabhängigen Druckwert, insbesondere eine Vorgabe für einen Zieldruck in der Füllungsphase und/oder eine Vorgabe für einen Zieldruck in der Entleerungsphase umfassen.

Gemäß einer weiteren Ausführungsform der Erfindung kann die Steuereinheit mindestens zwei Steuermodule umfassen, wobei das erste Steuermodul eingerichtet ist die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck zu bestimmen und eine Einlass- und/oder Auslassflussregelung durchzuführen, d.h. die Arbeitspumpe gemäß den vorgenannten Vorgaben so zu steuern, dass der vorgegebene Einlass- und/oder Auslassfluss im Wesentlichen erreicht wird. Das zweite Steuermodul kann eingerichtet sein, die Positions- und die Druckvorgabe zu bestimmen und entsprechend eine Positions- und Druckregelung durchzuführen, d.h. die Arbeitspumpe gemäß der Positions- und Druckvorgabe so zu steuern, dass die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck im Wesentlichen erreicht werden. Alternativ ist es auch denkbar, dass das erste Steuermodul eingerichtet ist, an Stelle des zweiten Steuermoduls die Positions- und Druckvorgabe zu bestimmen und diese an das zweite Steuermodul zur Positions- und Druckregelung weiterzuleiten.

Der Arbeitskolben kann vorzugsweise mittels eines elektrischen Spindelmotors angetrieben werden.

In einer weiteren vorteilhaften Ausgestaltung der Erfindung kann die erste Arbeitspumpe einen geschlossenen Pneumatikschaltkreis (im Folgenden auch als Kompressorpumpe bezeichnet) umfassen, in dem ein Kompressor, ein Unterdruckbehälter, ein Arbeitspumpenventil und ein Überdruckbehälter der Reihe nach angeordnet sind, wobei das Arbeitspumpenventil derart steuerbar ist, dass über das Arbeitspumpenventil abwechselnd ein im Unterdruckbehälter anliegender Unterdruck und ein im Überdruckbehälter anliegender Überdruck auf die erste Druckleitung übertragen wird, und die Steuereinheit eingerichtet ist, basierend auf dem erfassten ersten Einlass- und/oder ersten Auslassfluss und/oder basierend auf der Vorgabe für den ersten Einlass- und/oder Auslassfluss eine Vorgabe für einen Mitteldruck im Arbeitsraum, eine Vorgabe für eine Druckamplitude im Arbeitsraum, eine Vorgabe für eine Pumprate, eine Vorgabe für eine relative Entleerungsdauer, eine Vorgabe für einen Entleerungsdruck und/oder eine Vorgabe für einen Füllungsdruck im Arbeitsraum zu bestimmen, und das Arbeitspumpenventil gemäß der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck so zu steuern, dass ein vorgegebener erster Einlass- und/oder Auslassfluss im Wesentlichen erreicht wird.

Unter dem Arbeitspumpenventil wird insbesondere ein 3/2-Wege-Ventil verstanden, welches insbesondere ein Stetigventil und/oder ein Proportionalventil sein kann. Durch das Arbeitspumpenventil kann bei der Übertragung des Überdrucks und des Unterdrucks ein Arbeitsfluid strömen, wobei ein Volumenstrom des Arbeitsfluids einstellbar ist. Durch eine Einstellbarkeit des Volumenstroms ermöglicht das Arbeitspumpenventil eine feinere und genauere Steuerung und Regelung des Drucks in der Druckleitung.

Unter Steuern des Arbeitspumpenventils gemäß den vorstehend genannten Vorgaben wird hier insbesondere eine Regelung zum Erreichen des vorgegebenen ersten Einlass- und/oder ersten Auslassflusses verstanden, wobei der erste Einlass- und/oder erste Auslassfluss Regelgrößen darstellen können und entsprechend der Mitteldruck, die Druckamplitude, die Pumprate, die relative Entleerungsdauer, den Entleerungsdruck und/oder der Füllungsdruck Stellgrößen darstellen können.

Weiterhin kann die Steuereinheit eingerichtet sein, aus der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck eine Druckvorgabe für die Druckleitung zu bestimmen und das Arbeitspumpenventil gemäß der Druckvorgabe so zu steuern, dass die Vorgabe für den Mitteldruck, die Vorgabe für die Druckamplitude, die Vorgabe für die Pumprate, die Vorgabe für die relative Entleerungsdauer, die Vorgabe für den Entleerungsdruck und/oder die Vorgabe für den Füllungsdruck im Wesentlichen erreicht wird. Unter Steuern des Arbeitspumpenventils gemäß den vorstehend genannten Vorgaben wird hier insbesondere eine Regelung zum Erreichen der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck verstanden, wobei der Druck eine Regelgröße darstellen kann und der Mitteldruck, die Druckamplitude, die Pumprate, die relative Entleerungsdauer, der Entleerungsdruck und der Füllungsdruck Stellgrößen darstellen können.

Die Druckvorgabe für die Druckleitung kann eine Vorgabe für ein Zeitverhalten des Drucks, eine Vorgabe für einen zeitabhängigen Druckwert oder eine Vorgabe für einen positionsabhängigen Druckwert, insbesondere eine Vorgabe für einen Zieldruck in der Füllungsphase und/oder eine Vorgabe für einen Zieldruck in der Entleerungsphase umfassen.

Wie bei der Kolbenpumpe als Arbeitspumpe kann auch bei der Kompressorpumpe die Steuereinheit mindestens zwei Steuermodule umfassen, wobei das erste Steuermodul eingerichtet ist die Vorgabe für die Druckamplitude, die Vorgabe für die Pumprate, die Vorgabe für die relative Entleerungsdauer, die Vorgabe für den Entleerungsdruck und/oder die Vorgabe für den Füllungsdruck zu bestimmen und eine Einlass- und/oder Auslassflussregelung durchzuführen, d.h. die Arbeitspumpe gemäß den vorgenannten Vorgaben so zu steuern, dass der vorgegebene Einlass- und/oder Auslassfluss im Wesentlichen erreicht wird. Das zweite Steuermodul kann eingerichtet sein, die Druckvorgabe zu bestimmen und eine Druckregelung durchzuführen, d.h. die Arbeitspumpe gemäß der Druckvorgabe so zu steuern, dass die Vorgabe für die Druckamplitude, die Vorgabe für die Pumprate, die Vorgabe für die relative Entleerungsdauer, die Vorgabe für den Entleerungsdruck und/oder die Vorgabe für den Füllungsdruck im Wesentlichen erreicht werden. Alternativ ist es auch denkbar, dass das erste Steuermodul eingerichtet ist, an Stelle des zweiten Steuermoduls die Druckvorgabe zu bestimmen und diese an das zweite Steuermodul zur Druckregelung weiterzuleiten.

Als Arbeitsmedium in der Arbeitspumpe kann insbesondere Luft verwendet werden. In diesem Fall kann, im Falle einer Kolbenpumpe als Arbeitspumpe, ein Druckaustausch zwischen dem Arbeitsraum, bzw., im Falle eines oben beschriebenen Pneumatikschaltkreises mit einem Kompressor, zwischen dem Unterdruck- oder Überdruckbehälter und der Druckleitung, zusammen mit einem Luftaustausch erfolgen.

Das erfindungsgemäße Pumpensystem kann weiterhin eine zweite Membranfluidpumpe, eine mit der zweiten Membranfluidpumpe verbundene zweite Einlasskanüle zum Zuleiten des Fluids zur zweiten Membranfluidpumpe sowie eine mit der zweiten Membranfluidpumpe verbundene zweite Auslasskanüle zum Ableiten des Fluids aus der zweiten Membranfluidpumpe , eine zweite Arbeitspumpe, welche über eine zweite Druckleitung mit der zweiten Membranfluidpumpe verbunden ist und eingerichtet ist, über die zweite Druckleitung die zweite Membranfluidpumpe anzutreiben, und einen zweiten Einlass- und/oder zweiten Auslassflusssensor zum Erfassen eines zweiten Einlassflusses in der zweiten Einlasskanüle und/oder eines zweiten Auslassflusses in der zweiten Auslasskanüle umfassen. In diesem Fall ist die Steuereinheit eingerichtet, die erste und/oder zweite Arbeitspumpe basierend auf dem erfassten ersten und/oder zweiten Einlass- und/oder Auslassfluss zu steuern.

Ferner kann das Pumpensystem auch eine Steuereinheit umfassen, die eine Vielzahl an Untereinheiten aufweist, wobei die Untereinheiten miteinander kommunizieren können. Insbesondere kann die Steuereinheit eine erste Untereinheit aufweisen, die eingerichtet ist, die erste Arbeitspumpe basierend auf dem erfassten ersten und/oder zweiten Einlass- und/oder Auslassfluss zu steuern, und eine zweite Untereinheit aufweisen, die eingerichtet ist, die zweite Arbeitspumpe basierend auf dem erfassten ersten und/oder zweiten Einlass- und/oder Auslassfluss zu steuern. Jede der Untereinheiten kann weiterhin ein oben beschriebenes erstes und ein oben beschriebenes zweites Steuermodul aufweisen.

Der erste und/oder zweite Einlass- und/oder Auslassflusssensor kann insbesondere einen Ultraschallsensor umfassen. Der erste und/oder zweite Einlass- und/oder Auslassflusssensor kann einen Ultraschallsensor umfassen, welcher insbesondere eingerichtet ist, den Einlass- und/oder Auslassfluss mittels eines Laufzeitverfahrens zu messen. Der Einlass- und/oder Auslassflussensor kann weiterhin derart ausgebildet sein, dass dieser alle Frequenzanteile des gemessenen Einlass- und/oder Auslassflusses erfasst. Der Einlassflusssensor kann derart an der Einlasskanüle angeordnet sein, dass dieser die Einlasskanüle umschließt, insbesondere vollständig umschließt. Der Auslassflusssensor kann an der Auslasskanüle so angeordnet sein, dass dieser die Auslasskanüle umschließt, insbesondere vollständig umschließt.

Die vorliegende Erfindung beinhaltet weiterhin ein Herzunterstützungssystem umfassend ein vorbeschriebenes Pumpensystem zum Pumpen von Blut, wobei die erste und/oder zweite Einlasskanüle mit einer Herzkammer und/oder einem Vorhof eines Herzens und die erste und/oder zweite Auslasskanüle mit einem Blutgefäß fluidisch verbindbar sind.

Die Erfindung umfasst auch ein Verfahren zum Betrieb eines vorstehend beschriebenen Pumpensystems, wobei die erste und/oder zweite Arbeitspumpe basierend auf dem erfassten ersten und/oder zweiten Einlass- und/oder Auslassfluss gesteuert wird.

Das Pumpensystem kann als stationäres oder mobiles System ausgebildet sein sowie Teil eines stationären oder mobilen Herzunterstützungssystem sein. Die vorstehend aufgeführten Merkmale des Pumpensystems können auch das Verfahren zum Betrieb des Pumpensystems vorteilhaft weiterbilden und umgekehrt.

Im Folgenden werden ein erfindungsgemäßes Pumpensystem, ein erfindungsgemäßes Herzunterstützungssystem sowie ein Verfahren zum Betrieb des Pumpensystems anhand konkreter Ausführungsbeispiele und unter Verwendung von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine schematische Ansicht eines Teils eines erfindungsgemäßen Herzunterstützungssystems,
- Figur 2: eine schematische Ansicht eines Teils eines ersten Ausführungsbeispiels eines erfindungsgemäßen Pumpensystems,
- Figur 3: ein erstes Versuchsbeispiel zur Veranschaulichung der Funktionsweise des erfindungsgemäßen Pumpensystems gemäß erstem Ausführungsbeispiel,
- Figur 4: ein zweites Versuchsbeispiel zur Veranschaulichung der Funktionsweise des erfindungsgemäßen Pumpensystems gemäß erstem Ausführungsbeispiel,
- Figur 5: ein drittes Versuchsbeispiel zur Veranschaulichung der Funktionsweise des erfindungsgemäßen Pumpensystems gemäß erstem Ausführungsbeispiel,
- Figur 6: eine schematische Ansicht eines Teils eines zweiten Ausführungsbeispiels eines erfindungsgemäßen Pumpensystems
- Figur 7: eine schematische Übersicht über eine erfindungsgemäße Verarbeitung durch eine erfindungsgemäße Steuereinheit gemäß dem ersten Ausführungsbeispiel und
- Figur 8: eine schematische Übersicht über eine erfindungsgemäße Verarbeitung durch eine erfindungsgemäße Steuereinheit gemäß dem zweiten Ausführungsbeispiel.

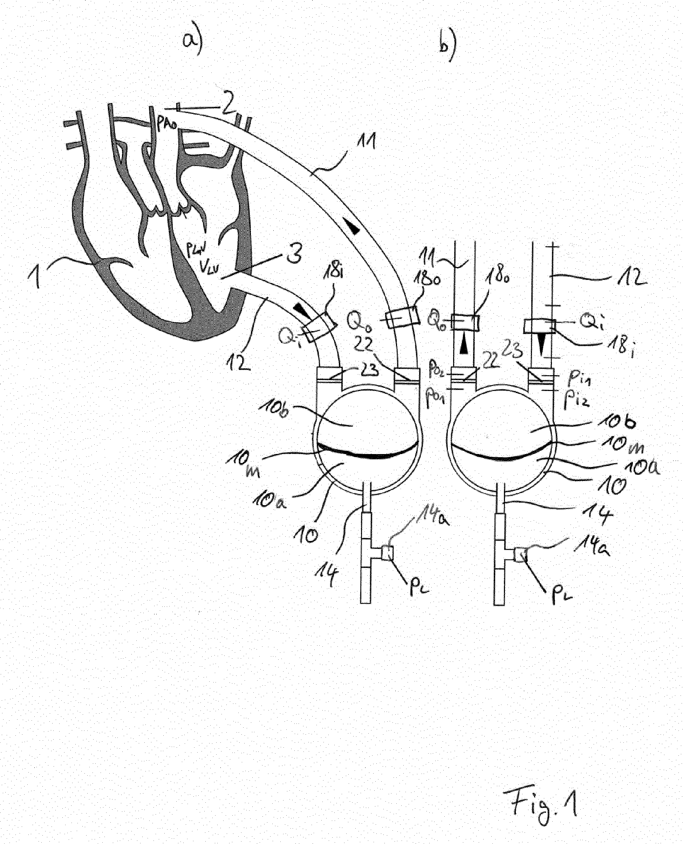

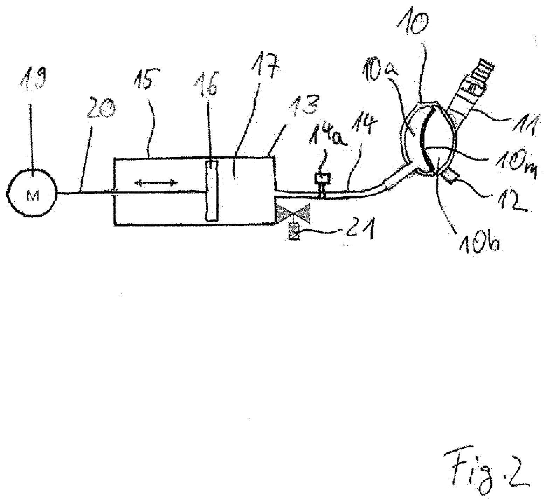

Figuren 1 und 2 zeigen schematische Ansichten von Teilen eines erfindungsgemäßen Herzunterstützungssystems.

Figur 1 zeigt einen Teil eines erfindungsgemäßen Herzunterstützungssystems. Figur 1 a) zeigt das Herzunterstützungssystem, wie es an einem menschlichen Herzen 1 angeordnet ist. Figur 1 b) zeigt das Herzunterstützungssystem mit weiteren Details. Das Herzunterstützungssystem umfasst eine Membranblutpumpe 10 mit einer im Inneren der Membranblutpumpe 10 angeordneten Membran 10m, welche das Innere der Membranblutpumpe 10 in eine Luftkammer 10a und eine Blutkammer 10b unterteilt. Das Herzunterstützungssystem umfasst weiterhin eine mit der Blutkammer 10b fluidisch verbundene Einlasskanüle 12, eine mit der Blutkammer 10b fluidisch verbundene Auslasskanüle 11 und eine mit der Luftkammer 10a fluidisch verbundene Druckleitung 14 auf. Die Blutkammer 10b der Membranblutpumpe 10 ist über die Einlasskanüle 12 mit der linken Herzkammer 3 des Herzens 1 und über die Auslasskanüle 11 mit einer Aorta 2 fluidisch verbindbar. Über die Druckleitung 14 ist die Luftkammer 10a der Membranblutpumpe mit einer Arbeitspumpe, beispielsweise einer Kolbenpumpe oder einer Kompressorpumpe (in Figur 1 nicht dargestellt) fluidisch verbindbar. In der Druckleitung 14 ist ferner ein Drucksensor 14a zum Messen eines in der Druckleitung 14 anliegenden Drucks angeordnet. An der Verbindungsstelle zwischen der Membranblutpumpe 10 und der Einlasskanüle 12 weist die Membranblutpumpe 10 ein Einlassventil 23 auf. An der Verbindungsstelle zwischen der Membranblutpumpe 10 und der Auslasskanüle 12 weist die Membranblutpumpe 10 ein Auslassventil 22 auf. Weiterhin ist an der Einlasskanüle 12 ein Einlassflusssensor 18i zum Messen eines Einlassflusses Qi angeordnet. Analog ist an der Auslasskanüle 11 ein Auslassflusssensor 18o zum Messen eines Auslassflusses Qo angeordnet.

Figur 2 zeigt ein erstes Ausführungsbeispiel eines erfindungsgemäßen Pumpensystems, welches in dem in Figur 1 gezeigten Herzunterstützungssystem eingesetzt werden kann. Das Pumpensystem umfasst eine wie in Figur 1 gezeigte Membranblutpumpe 10 sowie eine mit der Membranblutpumpe 10 fluidisch verbundene Druckleitung 14 auf. Das Pumpensystem weist weiterhin eine Kolbenpumpe 15 als Arbeitspumpe zum Antreiben der Membranblutpumpe 10 auf. Die Kolbenpumpe 15 umfasst ein hohlzylinderförmiges Gehäuse 13, in dem ein Arbeitskolben 16 oszillatorisch entlang der Längsachse des hohlzylinderförmigen Gehäuses 13 hin und her bewegbar ist. Der Arbeitskolben 16 kann dabei so bewegt werden, dass sich ein in dem hohlzylinderförmigen Gehäuse 15 befindlicher Arbeitsraum 17 oszillatorisch verkleinert und vergrößert. Die oszillatorische Bewegung des Arbeitskolbens 16 wird dabei durch eine von einem Elektromotor 19 angetriebene und sowohl gegen als auch im Uhrzeigersinn drehbare Spindel 20 vermittelt. Als Spindel 20 kann auch eine Kugelgewindespindel eingesetzt werden. In einer Wandung des hohlzylinderförmigen Gehäuses 15 ist weiterhin ein Ausgleichsventil 21 angeordnet, zum Ausgleich einer Menge des sich im Arbeitsraum 17 befindlichen Arbeitsmediums. Als Arbeitsmedium wird hier Luft verwendet. In der Druckleitung 14 ist ein Drucksensor 14a angeordnet. Dieser kann jedoch auch als Teil der Kolbenpumpe 15 im Arbeitsraum 17 angeordnet sein.

Wie bereits in Figur 1 gezeigt ist, weist die Membranblutpumpe 10 eine flexible Membran 10m auf, welche die Membranblutpumpe in eine Blutkammer 10b und eine Luftkammer 10a unterteilt. Der Arbeitsraum 17 der Kolbenpumpe 15 steht in einer Druck- und Stoffaustauschverbindung mit der Druckleitung 14 und der Luftkammer 10a, sodass sich die Luft zwischen dem Arbeitskolben 16 und der Membran 10m ausbreiten kann. Die Blutkammer 10b steht über die Membran 10m in einer Druckaustauschverbindung mit der Luftkammer 10a, der Druckleitung 14, dem Arbeitsraum 17 und den Ein- und Auslasskanülen 12 und 11, sodass sich ein Druck im Arbeitsraum 17 auf die Ein- und Auslasskanülen 12 und 11 auswirken kann. Weiterhin steht die Blutkammer 10b in einer Stoffaustauschverbindung mit den Ein- und Auslasskanülen 12 und 11 und dem Herz-Kreislauf-System, sodass Blut je nach Öffnungszustand der Ventile 23 und 22 von der linken Herzkammer 3 über die Einlasskanüle 12 in die Blutkammer 10b einströmen und über die Auslasskanüle 11 aus der Blutkammer 10b heraus und in die Aorta 2 einströmen kann.

Im Folgenden wird die Arbeitsweise des Pumpensystems gemäß einem Ausführungsbeispiel beschrieben. Die Steuereinheit (in den Figuren nicht dargestellt) kann den Steuerstrom für den Motor 19 zum Antreiben der Spindel mit vorgeben. Dadurch bewegt sich der Arbeitskolben 16 und baut abwechselnd einen Entleerungs- und einen Füllungsdruck p_{L} mit der Maßgabe auf, dass der Einlass- und der Auslassfluss konstant bleiben und sich die Blutkammer 10b vollständig füllt und entleert. Der Entleerungsdruck und der Füllungsdruck p_{L} wirken abwechselnd auf die Membran 10b, die dadurch ebenfalls abwechselnd einen positiven und negativen Druck pₒ und pᵢ in der Ein- bzw. Auslasskanüle aufbaut. Im Falle des Entleerungsdrucks in der Entleerungsphase ist das Auslassventil 22 geöffnet und das Einlassventil 23 geschlossen, sodass Blut aus der Blutkammer 10b durch die Auslasskanüle 11 in die Aorta 2 gepumpt wird. Dabei misst der Auslassflusssensor 18o den Auslassfluss Qo über die gesamte Entleerungsdauer. Anschließend wird ein Füllungsdruck in der Füllungsphase aufgebaut, das Auslassventil 22 geschlossen und das Einlassventil 23 geöffnet, sodass Blut aus der linken Herzkammer 3 durch die Einlasskanüle 12 in die Blutkammer 10b fließen kann. Dabei misst der Einlassflusssensor 18i den Einlassfluss Qi über die gesamte Füllungsdauer. Der Auslassflussensor 18o misst außerdem einen Rückfluss während der Füllungsphase, und der Einlassflussensor misst einen Rückfluss in der Entleerungsphase. Die Flussmessungen jedes Pumpzyklus werden aufgezeichnet. Anhand des Zeitverhaltens des Ein- und Auslassflusses können dann beispielsweise von Pumpzyklus zu Pumpzyklus Laständerungen im System schnell erkannt und beispielsweise durch Anpassen des Mitteldrucks, der Druckamplitude und/oder der Pumprate schnell kompensiert werden.

Im Folgenden werden drei beispielhafte Versuche beschrieben, welche die Funktionsweise des erfindungsgemäßen Pumpensystems veranschaulichen.

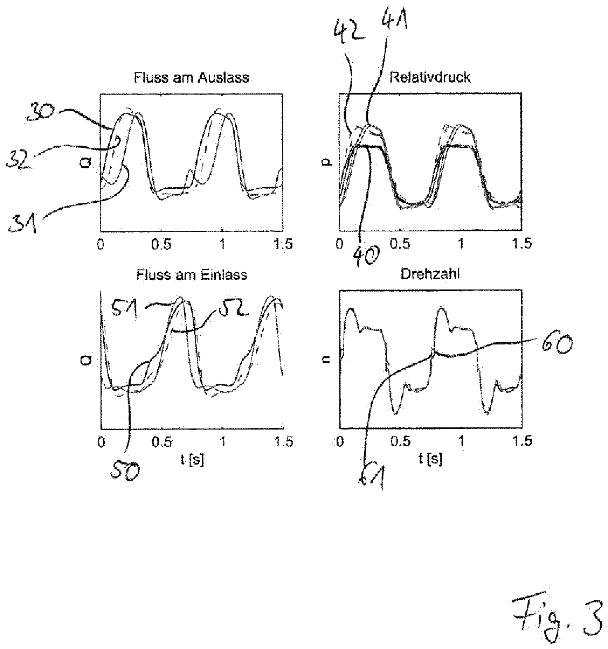

In einem ersten Versuch wurde der Druck p_{Ao} hinter der Auslasskanüle 11 erhöht, sodass der durch die Membran 10m erzeugte Druck pₒ einen Gegendruck erfährt und sich ein verringerter Druckunterschied von dem Auslassventil 22 zur Aorta 2 und dadurch ein verringerter Blutfluss Qo ergibt. Das Ergebnis des ersten Versuchs ist in Figur 3 dargestellt. Links oben ist das Zeitverhalten des Auslassblutflusses Qo am Blutflusssensor 18o dargestellt. Links unten ist das Zeitverhalten des Einlassblutflusses Qi am Blutflusssensor 18i dargestellt. Rechts oben ist das Zeitverhalten des im Arbeitsraum 17 herrschenden Drucks dargestellt. Rechts unten ist das Zeitverhalten der Drehzahl der Spindel 20 dargestellt.

Im Druckdiagramm entspricht Kurve 40 einem Referenz-Druck. Durch Verkleinern des Arbeitsraumes 17 steigt der Druck in der Luftkammer 10a, wodurch sich die Membran 10m bei Erreichen eines Ziel-Entleerungsdrucks zu den Kanülen 11 und 12 hin vorwölbt. Der Druck wird nun solange konstant gehalten bis der Arbeitsraum 17 zum Ende der vorgegebenen Entleerungsdauer wieder vergrößert wird. Danach fällt der Druck in der Luftkammer 10a bis in den negativen Bereich, sodass sich ein Füllungsdruck aufbaut und sich die Membran 10m bei einem entsprechenden Ziel-Füllungsdruck zur Druckleitung 14 hin vorwölbt. Der Füllungsdruck wird nun konstant gehalten bis sich der Arbeitsraum 17 zum Ende der vorgegebenen Füllungsdauer wieder vergrößert. Kurve 41 entspricht dem Zeitverhalten des Drucks mit p_{Ao}-Erhöhung. Kurve 42 entspricht einer Kompensation der Erhöhung von p_{Ao} durch manuelle Anpassung des Mitteldrucks im Arbeitsraum und des Hubs des Arbeitskolbens 16 durch Anpassen der Kolbenbewegung und der Luftmenge.

Im Diagramm des Auslassflusses entspricht Kurve 30 einem Referenz-Fluss. Dieser nimmt in der Entleerungsphase bis zu einem Maximalwert zu und nach etwa der Hälfte der Entleerungsdauer aufgrund der stetigen Entleerung der Blutkammer 10b wieder ab bis die Blutkammer 10b zum Ende der Entleerungsphase vollständig entleert ist. In der Füllungsphase ist der Auslassfluss nahezu null, da das Auslassventil 22 geschlossen ist. Kurve 31 entspricht dem Auslassfluss bei erhöhtem p_{Ao}. Es ist erkennbar, dass der Fluss erst später als bei Kurve 30 größer als null ist, da von der Membran 10m erst ein höherer Gegendruck zu p_{Ao} erzeugt werden muss, damit der Druckunterschied zwischen der Blutkammer 10b und der Auslasskanüle 11 groß genug ist, um das Auslassventil 22 zu öffnen. Relativ kurz nach Erreichen des Maximalflusses, der etwa dem Maximalfluss der Kurve 30 entspricht, nimmt der Fluss aufgrund des zum Ende der Entleerungsphase sinkenden Druckes p_{L} wieder ab bis auf etwa null in der Füllungsphase, in der das Auslassventil 22 geschlossen ist. Es ist somit anhand der schmaleren Auslassflusskurve 31 erkennbar, dass es aufgrund der p_{Ao}-Erhöhung nicht zu einer vollständigen Entleerung der Blutkammer 10b kommt. Kurve 32 stellt das Zeitverhalten des Auslassflusses nach manueller Anpassung des Mitteldruckes und des Hubs dar, welche in etwa der Referenz-Kurve 30 entspricht.

Im Diagramm des Einlassflusses stellt Kurve 50 einen Referenz-Fluss und Kurve 51 ein Zeitverhalten eines Flusses bei erhöhtem p_{Ao} dar. Aufgrund der p_{Ao}-Erhöhung erreichte die Membran 10m in der vorangegangenen Entleerungsphase nicht ihre vorgesehene Endlage, um die Blutkammer 10b vollständig zu entleeren. Aus diesem Grund ist auch der Bewegungsbereich der Membran 10m in der Füllungsphase bis zur vorgesehenen Endlage geringer. Dies hat einen geringeren Einlassfluss zur Folge, wie aus Kurve 51 im Vergleich zu Kurve 50 erkennbar ist. Kurve 52 entspricht wieder einem Zeitverhalten eines Flusses nach manueller Anpassung des Mitteldruckes und des Hubs.

Aus dem Drehzahl-Diagramm ist ersichtlich, dass die Druckerhöhung von pAO nur einen sehr geringen Unterschied im Zeitverhalten der Drehzahl bewirkt, da bereits eine Anpassung des Mitteldrucks ausreicht, um den Ausgangsfluss wieder herzustellen. Hier entspricht Kurve 60 einer Referenz-Drehzahl und Kurve 61 der Drehzahl bei Druckerhöhung. Die zusätzliche Huberhöhung kann gering ausfallen.

In Figur 3 wird also deutlich, dass die im Stand der Technik übliche Messgröße Druck nicht eindeutig den Flussrückgang erkennen lässt und es daher schwierig ist diesem sofort entgegen zu wirken. Bei der Auslassflussmessung hingegen ist sowohl am Mittelwert des Flusses als auch am Zeitverhalten des Flusses zu erkennen, wie stark die Antriebsleistung erhöht werden muss, um das ursprünglich eingestellte Entleerungsverhalten aufrecht zu erhalten. Es ist beispielsweise erkennbar, dass der Entleerungsdruck erhöht werden muss, weil der Auslassfluss später beginnt (und das der Füllungsdruck abgesenkt werden kann). Weiterhin ist anhand des reduzierten Flusses ersichtlich, dass eine Anpassung erforderlich ist. In diesem Fall würde ein Regler beispielsweise gleichzeitig den Mitteldruck erhöhen, um die ursprüngliche Symmetrie des Flusses wiederherzustellen, und den Kolbenhub erhöhen. Erst bei einem Mitteldruck, der den Fluss nicht mehr erhöht, würde der Kolbenhub beispielsweise wieder verringert werden.

In der Füllungsphase lässt sich bei der Auslassflussmessung außerdem die Dichtigkeit des Auslassventils (Rückströmung) erkennen und wie stark die Fluidsäule in der Kanüle zum Öffnungszeitpunkt des Ventils schwingt. Eine Zunahme der Schwingungsneigung könnte ein Anzeichen für zu geringe Drücke sein.

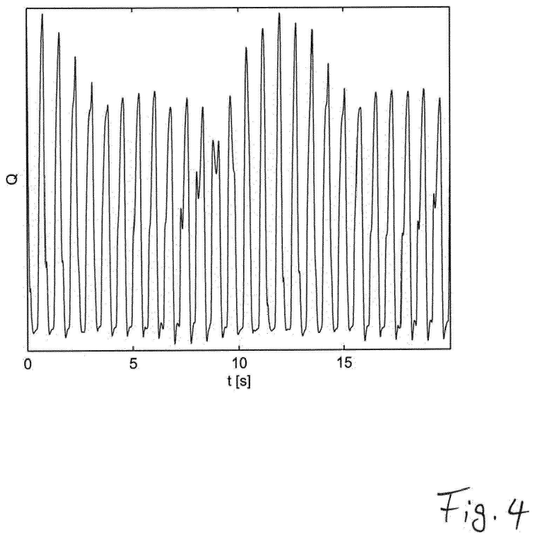

In einem zweiten Versuch wird der Druck p_{LV} (siehe Figur 1 a)) vor der Einlasskanüle 12 rechteckförmig geändert, um Druckschwankungen durch das Herz 1 zu simulieren. Das Ergebnis des zweiten Versuchs ist in Figur 4 dargestellt. Figur 4 zeigt das Zeitverhalten eines Einlassflusses Qi gemessen mit dem Einlassflusssensor 18i. Über die Messung von Qi lässt sich auf die Rate und die Amplitude der Störung von p_{LV} zurückschließen. Es ist somit prinzipiell möglich, diese Störung zu kompensieren oder ein festes Verhältnis von Herzrate zu Pumprate einzustellen. Unter der Annahme, dass die Herzrate bei einigen Patienten ein Indikator für den erforderlichen Blutvolumenstrom ist, könnte seine Zielgröße an den Bedarf angepasst werden.

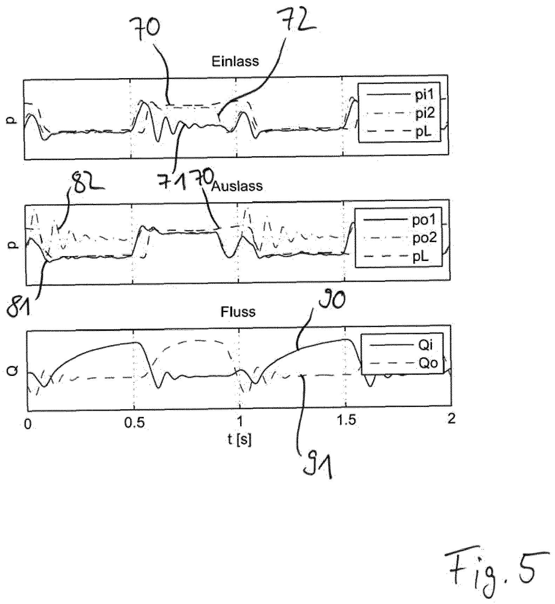

In einem dritten Versuch werden im Normalbetrieb der Druck p_{L}, der Einlassfluss Qi, der Auslassfluss Qo und weitere Drücke pᵢ₁ und pᵢ₂ vor und hinter dem Einlassventil 23 sowie weitere Drücke pₒ₁ und pₒ₂ vor und hinter dem Auslassventil 22 gemessen. Die genauen Messstellen der Drücke pᵢ₁, pᵢ₂, pₒ₁ und pₒ₂ sind in Figur 1 b) eingezeichnet. Die Ergebnisse des dritten Versuchs sind in Figur 5 dargestellt. Kurve 70 beschreibt das Zeitverhalten des Druckes p_{L}. Kurve 71 beschreibt das Zeitverhalten des Druckes pᵢ₁ vor dem Einlassventil 23. Kurve 72 beschreibt das Zeitverhalten des Druckes pᵢ₂ hinter dem Einlassventil 23. Kurve 81 beschreibt das Zeitverhalten des Druckes pₒ₁ vor dem Auslassventil 22. Kurve 82 beschreibt das Zeitverhalten des Druckes pₒ₂ hinter dem Auslassventil 22. Kurve 90 beschreibt das Zeitverhalten des Einlassflusses Qi und Kurve 91 beschreibt das Zeitverhalten des Auslassflusses Qo. Hierbei ist vor allem interessant, dass die Druckverhältnisse in der Membranblutpumpe 10 und das Ventilschließverhalten des Einlassventils 23 und des Auslassventils 22 mit der Druck-Messung von p_{L} nicht beobachtet werden können. Im Zeitverlauf des Blutflusses sind die Vorgänge besser zu erkennen.

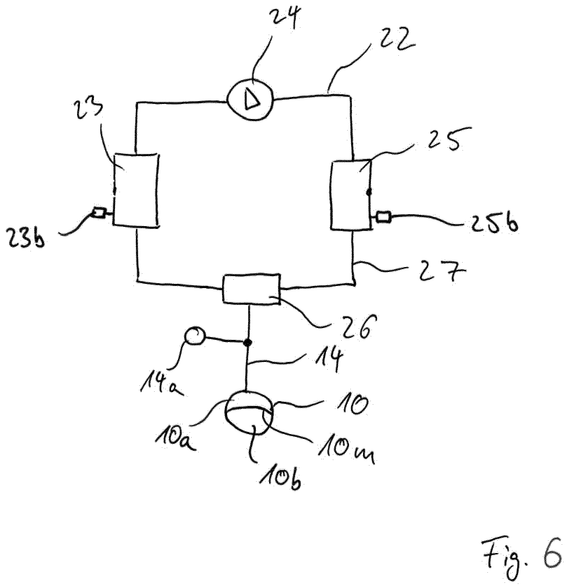

Figur 6 zeigt eine schematische Ansicht eines Teils eines zweiten Ausführungsbeispiels eines erfindungsgemäßen Pumpensystems, wobei lediglich für die vorliegende Erfindung wesentliche Bestandteile dargestellt sind. Das Pumpensystem in Figur 6 weist als Arbeitspumpe eine Kompressorpumpe auf. Die Kompressorpumpe umfasst einen Unterdruckbehälter 23, einen Kompressor 24, einen Überdruckbehälter 25 und ein Proportionalventil 26 als Arbeitspumpenventil, welche innerhalb eines geschlossenen Pneumatikschaltkreises 22 der Reihe nach über eine erste Druckleitung 27 miteinander verbunden sind. Weiterhin befindet sich das Proportionalventil 26 in einer fluidischen Verbindung mit einer zweiten Druckleitung 14. Die Druckleitung 14 ist ferner mit einer Membranblutpumpe 10 fluidisch verbunden, wobei die Membranblutpumpe 10 eine Membran 10m aufweist, welche ein Inneres der Membranblutpumpe 10 in eine mit der zweiten Druckleitung 14 verbundene Luftkammer 10a und eine sich auf einer der Luftkammer 10a gegenüberliegenden Seite der Membran 10m befindliche Blutkammer 10b teilt. Die Blutkammer 10b ist, wie in Figur 1 dargestellt, mit einer Einlasskanüle 12 und einer Auslasskanüle 11 verbunden. Weiterhin ist an der Einlasskanüle 11 ein Einlassflussensor 18i zum Erfassen eines Einlassflusses Qi und an der Auslasskanüle 11 ein Auslassflusssensor 18o zum Erfassen eines Auslassflusses Qo angeordnet. Die Ein- und Auslasskanülen 12 und 11 sowie die Ein- und Auslassflusssensoren 18i und 18o sind in Figur 6 nicht dargestellt. In der Druckleitung 14 ist ein Drucksensor 14a angeordnet. Dieser kann jedoch auch als Bestandteil der Kompressorpumpe ausgebildet sein.

Nachfolgend wird die Funktionsweise der Kompressorpumpe beschrieben. Der Kompressor 24 erzeugt einen Überdruck im Überdruckbehälter 25 und einen Unterdruck im Unterdruckbehälter 23. Mithilfe der Ventile 25b im Überdruckbehälter und 23b im Unterdruckbehälter kann der Überdruck im Überdruckbehälter 25 und der Unterdruck im Unterdruckbehälter 23 auf die vorbestimmten Druckwerte geregelt werden. Das Proportionalventil ist eingerichtet, je nach Öffnungszustand, den im Unterdruckbehälter 23 anliegenden Unterdruck oder den im Überdruckbehälter 25 anliegenden Überdruck auf die zweite Druckleitung 14 zu übertragen. Dabei kann der durch das Proportionalventil strömende Volumenstrom der Luft, also das Luftvolumen, welches innerhalb einer bestimmten Zeitspanne durch das Proportionalventil strömt, eingestellt werden. Durch das wechselseitige Übertragen des im Überdruckbehälter 25 anliegenden Überdrucks und des im Unterdruckbehälter anliegenden Unterdrucks entsteht in der zweiten Druckleitung 14 abwechselnd ein Entleerungsdruck und ein Füllungsdruck. Der Entleerungsdruck und der Füllungsdruck werden über die Luftkammer 10a der Membranblutpumpe 10 auf die Membran 10m der Membranblutpumpe 10 übertragen, welche Blut bei Anliegen des Entleerungsdrucks aus der Blutkammer 10b in die Auslasskanüle 11 drückt oder bei Anliegen des Füllungsdrucks aus der Einlasskanüle 12 in die Blutkammer 10b hinein saugt. Beim Durchströmen der Ein- und Auslasskanülen 12 und 11 kann der Fluss des Blutes erfasst werden und in vorbestimmten Zeitabständen die Kompressorpumpe basierend auf den erfassten Flüssen gesteuert werden.

Die wichtigsten Regelungsziele beim Betrieb des Pumpensystems sind:
- Die Membranblutpumpe muss vollständig entleert und gefüllt werden. Dabei ist unter vollständigem Füllen und Entleeren eine vollständige Bewegung der Membran in ihre Endlagen gemeint. Am Ende der Entleerungsphase kann ein Restblutvolumen, am Ende der Füllungsphase auf der Luftseite ein Restluftvolumen vorhanden sein.
- Ein nicht aktiv eingeleiteter Fluss-Rückgang soll sofort alarmiert und kompensiert werden.
- Die Herzkammer bzw. der Vorhof soll nicht zu stark entleert werden, um die Herzwand nicht anzusaugen.
- Die Füllungs- und Entleerungsdrücke sollen niedrig sein.
- Im Falle eines Betriebs mit zwei Membranblutpumpen (biventrikulärer Betrieb) soll der Blutfluss der rechten Membranblutpumpe nicht größer sein als der auf der linken Seite, um Lungenschäden zu vermeiden.
- Alle Hardware-Grenzen (beispielsweise für Kolbenpumpe: Drehzahl, Strom, Verfahrweg, ...) sollen eingehalten werden.
- Patienten- und systembedingte Varianzen sollen im biventrikulären Betrieb kompensiert werden.

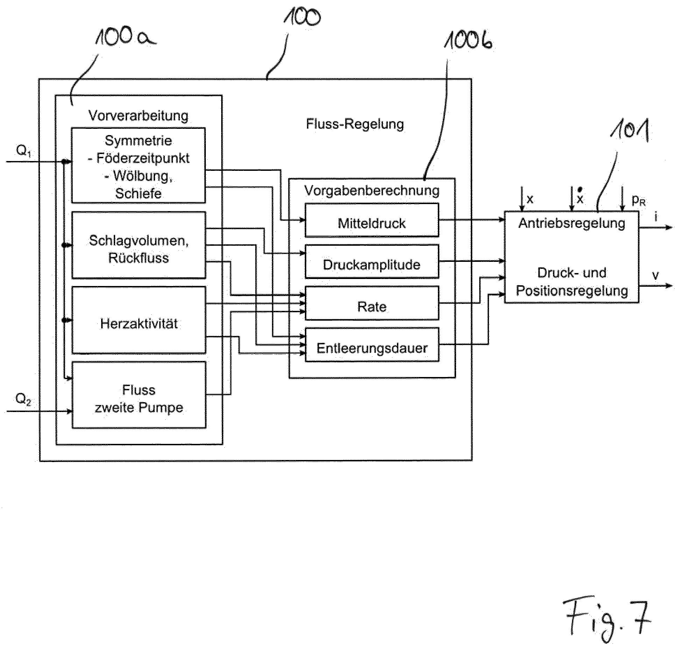

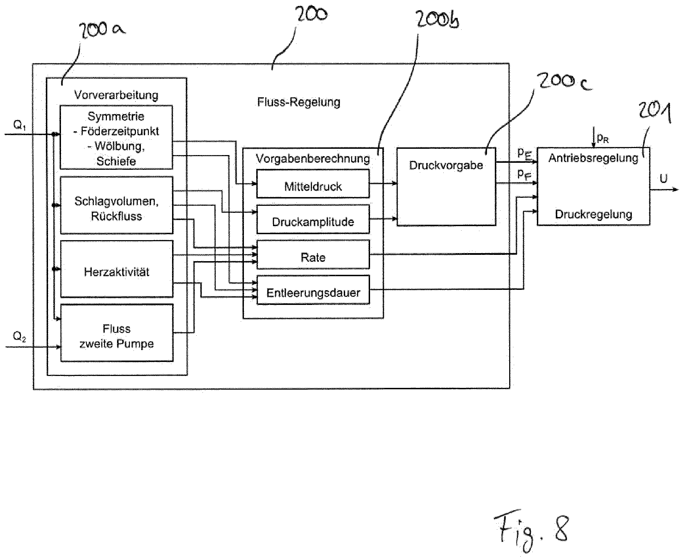

Diese Ziele können mit Hilfe der Flussmessung und -regelung besser erreicht werden. Einen Überblick über die wichtigsten Ausgestaltungsvarianten der Flussregelung gibt Figur 7 für eine Kolbenpumpe gemäß Figur 2 sowie Figur 8 für eine Kompressorpumpe gemäß Figur 6, sowohl für den Fall einer Membranblutpumpe als auch zweier Membranblutpumpen.

Zunächst wird die Flussregelung für die Kolbenpumpe anhand von Figur 7 beschrieben. Die Kolbenpumpe wird mittels einer Steuereinheit gesteuert. Die Steuereinheit umfasst ein erstes Steuermodul 100, welches die Flussregelung durchführt, und ein zweites Steuermodul 101, welches die Antriebsregelung durchführt. In dem ersten Steuermodul 100 wird zunächst in der Vorverarbeitungseinheit 100a eine Vorverarbeitung der Flussmesswerte durchgeführt. Hierbei werden aus den Messwerten Q1 und Q2 der Flüsse zweier Blutpumpen in je einer Kanüle Merkmale der Flüsse, wie z.B. Symmetrie (Förderzeitpunkt, Wölbung, Schiefe), Pumpvolumen, Rückfluss, Herzaktivität, Flussverhältnis und Flussdifferenz von Q1 und Q2 extrahiert. In der Vorgabenberechnungseinheit 100b werden aus diesen extrahierten Merkmalen Vorgaben beispielsweise für den Mitteldruck, die Druckamplitude, die Rate und die Entleerungsdauer berechnet. Die berechneten Vorgaben werden dann an das zweite Steuermodul 101 für die Antriebsregelung übermittelt. In dem zweiten Steuermodul 101 werden aus den Vorgaben der Vorgabenberechnungseinheit 100b sowie unter Berücksichtigung der Position und Geschwindigkeit des Arbeitskolbens 16 und des Drucks im Arbeitsraum 17 Vorgaben für die Kolbenpositionsregelung und die Druckregelung berechnet und daraus ein Steuerstrom i für den Spindelmotor 19 und eine Ventilöffnungsvorgabe v für das Ausgleichsventil 13 ermittelt.

Alternativ zur in Figur 7 gezeigten Regelungsstruktur ist es auch denkbar, dass die Berechnung der Druck- und Positionsvorgabe ebenfalls im ersten Steuermodul 100 erfolgt.

Figur 8 zeigt eine Flussregelungsstruktur im Falle der Kompressorpumpe. Die Kompressorpumpe wird mittels einer Steuereinheit gesteuert. Die Steuereinheit umfasst ein erstes Steuermodul 200, welches die Flussregelung durchführt, und ein zweites Steuermodul 201, welches die Antriebsregelung durchführt. Hierbei werden in einer Vorverarbeitungseinheit 200a wie im Falle der Kolbenpumpe aus den Messwerten Q1 und Q2 der Flüsse zweier Membranblutpumpen in je einer Kanüle Merkmale der Flüsse, wie z.B. Symmetrie (Förderzeitpunkt, Wölbung, Schiefe), Pumpvolumen, Rückfluss, Herzaktivität, Flussverhältnis und Flussdifferenz von Q1 und Q2 extrahiert. In einer ersten Vorgabenberechnungseinheit 200b werden aus diesen extrahierten Merkmalen Vorgaben beispielsweise für den Mitteldruck, die Druckamplitude, die Rate und die Entleerungsdauer berechnet. Aus den berechneten Vorgaben werden dann in einer zweiten Vorgabenberechnungseinheit 200c Druckvorgaben für einen Entleerungsdruck und einen Füllungsdruck berechnet. Diese Druckvorgaben werden an das zweite Steuermodul 201 übermittelt. In dem zweiten Steuermodul 201 wird aus den Druckvorgaben für den Entleerungsdruck und den Füllungsdruck sowie unter Berücksichtigung des Drucks in der zweiten Druckleitung 14 eine Steuerspannung für das Proportionalventil 26 berechnet.

Alternativ zur in Figur 8 gezeigten Regelungsstruktur ist es auch denkbar, dass die Berechnung der Druckvorgaben in dem zweiten Steuermodul 201 für die Antriebsregelung erfolgt. In diesem Fall kann auf eine zweite Vorgabenberechnungseinheit 200c im ersten Steuermodul 200 verzichtet werden.

Im Einzelnen können die Regelungsziele mit folgenden Maßnahmen erreicht werden:

### Vorverarbeitung

Symmetrie - Ein Symmetriewert S wird aus dem Zeitverhalten des gemessenen Flusses bestimmt. Dieser gibt entweder für die Messung des Flusses in einer Kanüle im Vergleich zu einem beim Einstellen ermittelten Referenz-Fluss die zeitliche Verschiebung des Spitzenflusses an oder vergleicht für zwei Kanülen die Symmetrie der zeitlichen Flussverläufe. In beiden Fällen könnte über den Symmetriewert der Mitteldruck so eingestellt werden, dass die Membranbewegung der Referenzbewegung beim Einstellen entspricht. Für die Berechnung des Symmetriewerts können neben der Verschiebung des zeitlichen Mittelpunkts weitere Merkmale wie die Wölbung und die Schiefe herangezogen werden.

Pumpvolumen und Rückfluss - Aus dem Flussverlauf lassen sich auch das Pumpvolumen und das Rückflussvolumen bestimmen. Es muss dazu eine Phase mit geöffnetem Einlass-/Auslassventil und eine Phase mit geschlossenem Einlass-/Auslassventil unterschieden werden. Das Rückflussvolumen ist das Produkt aus der Dauer eines Pumpzyklus und dem über die Phase mit geschlossenem Einlass-/Auslassventil gemittelten Fluss. Das Pumpvolumen ist das Zeitintegral des Flusses über die Phase mit geöffnetem Einlass-/Auslassventil. Mit dem Pumpvolumen lässt sich die erforderliche Druckamplitude bestimmen, mit dem Rückflussvolumen kann die Rate angepasst werden. Wenn das Pumpvolumen nicht auf erhöhte Druckamplituden reagiert, kann ebenfalls die Rate angepasst werden. Es ist auch denkbar die Entleerungsdauer anzupassen, um das Pumpvolumen konstant zu halten.

Herzaktivität - Bei der Auswertung des Flusses bzgl. Herzaktivität sind zwei Merkmale besonders wichtig und gut zu erkennen: Die Amplitude und die Rate. Die Rate kann in Form eines Ratenverhältnisses zur Anpassung der Antriebsrate verwendet werden. Die Amplitude der Flussstörung kann eingesetzt werden, um die Druckamplitude so klein wie möglich und so groß wie nötig zu halten.

Vergleich mit Fluss einer zweiten Membranblutpumpe - Beim Vergleich mit der anderen Membranblutpumpe können im biventrikulären Pumpbetrieb entweder die Flussdifferenz oder das Flussverhältnis beider Seiten bestimmt werden. Diese Größen können dann durch Ratenanpassungen konstant gehalten werden.

Äußere Regelkreise - Für den äußeren Regelkreis ergeben sich also mindestens folgende Teile:
Mitteldruckregelung - Der Regler für den Mitteldruck berechnet aus einer Referenz für den Symmetriewert S_{R}, die beim Einstellen des Antriebs ermittelt wird, und S aus den letzten Pumpzyklen zunächst die Abweichung e = S_{R} - S. Er passt dann in jedem Pumpzyklus den Mitteldruck an, um e zu reduzieren.

Druckamplitude - Der Regler für die Druckamplitude beinhaltet eine Anpassung an das Pumpvolumen und/oder eine Anpassung an die Herzaktivität. Die Anpassung an das Pumpvolumen erfolgt analog zur Mitteldruckregelung mit Hilfe einer beim Einstellen ermittelten Referenz. Die Anpassung an die Herzaktivität kann aus einer Erhöhung der Druckamplitude bei zunehmender Amplitude der Flussschwankung bestehen oder dynamisch erfolgen. Wenn die Auswurfphase des Herzens mit der Füllungsphase der Membranblutpumpe zusammenfällt, wird die Druckamplitude abgesenkt. Wenn einige Pumpzyklen später beide Phasen versetzt sind, wird der Druck wieder erhöht. Für die zuletzt genannte Regelung bietet sich als Stellgröße statt der Druckamplitude auch der Füllungsdruck an, da eine Anpassung der Entleerungsphase kaum nötig ist.

Ratenregler - Die Ratenanpassung sollte den Rückfluss kompensieren, der Herzrate des Patienten folgen und sicherstellen, dass die rechte Membranblutpumpe im biventrikulären Betrieb stets weniger Unterstützung bereitstellt, als die an die linke Herzhälfte angeschlossene Membranblutpumpe.

Innerer Regelkreis - Im Falle einer Kolbenpumpe als Arbeitspumpe können für den inneren Regelkreis eine Druck- und/oder Positionsregelung verwendet werden. Im Falle der Kompressorpumpe kann für den inneren Regelkreis eine Druckregelung verwendet werden. Eine Druckregelung ist vorteilhaft, weil sich hier die Vorgaben der äußeren Regler schneller umsetzen lassen. Das erhöht theoretisch die Geschwindigkeit und die Stabilitätsreserven der Verfahren. Ein Beispiel für einen sehr schnellen Regler ist das Halten eines Drucks, bis ein bestimmtes Pumpvolumen erreicht ist oder bis der Fluss auf einen Grenzwert zurückgegangen ist. Hier würde die Anpassung der Rate und der Entleerungsdauer innerhalb eines Pumpzyklus erfolgen und im nächsten Pumpzyklus könnte mit einer Druckanpassung reagiert werden.

## Patentansprüche

1. Pumpensystem zum Pumpen eines Fluids, wobei das Pumpensystem umfasst:
eine erste Membranfluidpumpe,
eine mit der ersten Membranfluidpumpe verbundene erste Einlasskanüle zum Zuleiten eines Fluids zur ersten Membranfluidpumpe sowie eine mit der ersten Membranfluidpumpe verbundene erste Auslasskanüle zum Ableiten des Fluids aus der ersten Membranfluidpumpe,
eine erste Arbeitspumpe,
welche über eine erste Druckleitung mit der ersten Membranfluidpumpe verbunden ist und eingerichtet ist, über die erste Druckleitung die erste Membranfluidpumpe anzutreiben,
**gekennzeichnet durch**
einen ersten Einlassflusssensor zum Erfassen eines ersten Einlassflusses des Fluids in der ersten Einlasskanüle und/oder einen ersten Auslassflusssensor zum Erfassen eines ersten Auslassflusses des Fluids in der ersten Auslasskanüle.

2. Pumpensystem nach dem vorhergehenden Anspruch, **gekennzeichnet durch** eine Steuereinheit zum Steuern der ersten Arbeitspumpe basierend auf dem erfassten ersten Einlass- und/oder Auslassfluss, insbesondere um eine Vorgabe für den ersten Einlass- und/oder Auslassfluss zu erreichen.

3. Pumpensystem nach dem vorhergehenden Anspruch, **dadurch gekennzeichnet, dass** die Vorgabe für den ersten Einlass- und/oder Auslassfluss eine Vorgabe für einen zeitlichen Mittelwert oder ein Zeitverhalten des ersten Einlass- und/oder Auslassflusses umfasst.

4. Pumpensystem nach Anspruch 2, **dadurch gekennzeichnet, dass** die Steuereinheit eingerichtet ist, basierend auf dem erfassten ersten Einlass- und/oder ersten Auslassfluss eine Vorgabe für einen in der ersten Druckleitung anliegenden Mitteldruck, eine Vorgabe für eine in der ersten Druckleitung anliegende Druckamplitude, eine Vorgabe für eine Pumprate der ersten Arbeitspumpe, eine Vorgabe für eine relative Entleerungsdauer, eine Vorgabe für einen in der ersten Druckleitung anliegenden Entleerungsdruck und/oder eine Vorgabe für einen in der ersten Druckleitung anliegenden Füllungsdruck zu bestimmen, und die erste Arbeitspumpe gemäß der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck so zu steuern, dass ein vorgegebener erster Einlass- und/oder erste Auslassfluss im Wesentlichen erreicht wird.

5. Pumpensystem nach Anspruch 2, **dadurch gekennzeichnet, dass** die Arbeitspumpe einen in einem Arbeitsraum oszillatorisch bewegbaren Arbeitskolben und ein Ausgleichsventil zum Ändern einer Masse eines Arbeitsfluids im Arbeitsraum und/oder eines Drucks im Arbeitsraum umfasst, wobei sich der Arbeitsraum in einer Druckaustauschverbindung mit der ersten Druckleitung befindet, und die Steuereinheit eingerichtet ist, basierend auf dem erfassten ersten Einlass- und/oder ersten Auslassfluss eine Vorgabe für einen Mitteldruck im Arbeitsraum, eine Vorgabe für eine Druckamplitude im Arbeitsraum, eine Vorgabe für eine Pumprate, eine Vorgabe für einen Hub des Arbeitskolbens, eine Vorgabe für eine Kraft des Arbeitskolbens, eine Vorgabe für eine relative Entleerungsdauer, eine Vorgabe für einen Entleerungsdruck und/oder eine Vorgabe für einen Füllungsdruck im Arbeitsraum zu bestimmen, und die Bewegung des Arbeitskolbens und das Ausgleichsventil gemäß der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck so zu steuern, dass ein vorgegebener erster Einlass- und/oder erster Auslassfluss im Wesentlichen erreicht wird.

6. Pumpensystem nach dem vorhergehenden Anspruch, **dadurch gekennzeichnet, dass** die Steuereinheit eingerichtet ist, basierend auf der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck eine Positionsvorgabe für den Arbeitskolben zu bestimmen, und die Bewegung des Arbeitskolbens gemäß der Positionsvorgabe und das Ausgleichsventil gemäß der Vorgabe für den Mitteldruck so zu steuern, dass die Vorgabe für den Mitteldruck, die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck im Wesentlichen erreicht wird.

7. Pumpensystem nach Anspruch 5, **dadurch gekennzeichnet, dass** die Steuereinheit eingerichtet ist, basierend auf der Vorgabe für den Mitteldruck, der Vorgabe für die Druckamplitude, der Vorgabe für die Pumprate, der Vorgabe für den Hub, der Vorgabe für die Kraft, der Vorgabe für die relative Entleerungsdauer, der Vorgabe für den Entleerungsdruck und/oder der Vorgabe für den Füllungsdruck eine Druckvorgabe für den Arbeitsraum und eine Vorgabe für eine Umkehrposition des Arbeitskolbens zu bestimmen, und die Bewegung des Arbeitskolbens gemäß der Druckvorgabe und das Ausgleichsventil gemäß der Vorgabe für die Umkehrposition so zu steuern, dass die Vorgabe für den Mitteldruck, die Vorgabe für die Druckamplitude, die Vorgabe für die Pumprate, die Vorgabe für den Hub, die Vorgabe für die Kraft, die Vorgabe für die relative Entleerungsdauer, die Vorgabe für den Entleerungsdruck und/oder die Vorgabe für den Füllungsdruck im Wesentlichen erreicht wird.

8. Pumpensystem nach dem vorhergehenden Anspruch, **dadurch gekennzeichnet, dass** der Arbeitskolben mittels eines elektrischen Spindelmotors antreibbar ist.

9. Pumpensystem nach Anspruch 2, **dadurch gekennzeichnet, dass** die erste Arbeitspumpe einen geschlossenen Pneumatikschaltkreis umfasst, in dem ein Kompressor, ein Unterdruckbehälter, ein Arbeitspumpenventil und ein Überdruckbehälter der Reihe nach angeordnet sind, wobei das Arbeitspumpenventil derart steuerbar ist, dass über das Arbeitspumpenventil abwechselnd ein im Unterdruckbehälter anliegender Unterdruck und ein im Überdruckbehälter anliegender Überdruck auf die erste Druckleitung übertragen wird, und die Steuereinheit eingerichtet ist, basierend auf dem erfassten ersten Einlass- und/oder ersten Auslassfluss eine Vorgabe für einen Mitteldruck im Arbeitsraum, eine Vorgabe für eine Druckamplitude im Arbeitsraum, eine Vorgabe für eine Pumprate, eine Vorgabe für eine relative Entleerungsdauer, eine Vorgabe für den Entleerungsdruck und/oder eine Vorgabe für einen Füllungsdruck im Arbeitsraum zu bestimmen, und das Arbeitspumpenventil gemäß der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck so zu steuern, dass ein vorgegebener erster Einlass- und/oder Auslassfluss im Wesentlichen erreicht wird.

10. Pumpensystem nach dem vorhergehenden Anspruch, **dadurch gekennzeichnet, dass** die Steuereinheit eingerichtet ist, basierend auf der Vorgabe für den Mitteldruck, der Vorgabe für die Druckamplitude, der Vorgabe für die Pumprate, der Vorgabe für die relative Entleerungsdauer, der Vorgabe für den Entleerungsdruck und/oder der Vorgabe für den Füllungsdruck eine Druckvorgabe für die Druckleitung zu bestimmen und das Arbeitspumpenventil gemäß der Druckvorgabe so zu steuern, dass die Vorgabe für den Mitteldruck, die Vorgabe für die Druckamplitude, die Vorgabe für die Pumprate, die Vorgabe für die relative Entleerungsdauer, die Vorgabe für den Entleerungsdruck und/oder die Vorgabe für den Füllungsdruck im Wesentlichen erreicht wird.

11. Pumpensystem nach einem der zwei vorhergehenden Ansprüche, **dadurch gekennzeichnet, dass** das Arbeitspumpenventil ein 3/2-Wegeventil, ein Stetigventil und/oder Proportionalventil ist, durch welches bei der Übertragung des Überdrucks und des Unterdrucks ein Arbeitsfluid strömt, wobei in dem Arbeitspumpenventil ein Volumenstrom des Arbeitsfluids einstellbar ist.

12. Pumpensystem nach einem der Ansprüche 2 bis 11, **gekennzeichnet durch**
eine zweite Membranfluidpumpe,
eine mit der zweiten Membranfluidpumpe verbundene zweite Einlasskanüle zum Zuleiten des Fluids zur zweiten Membranfluidpumpe sowie eine mit der zweiten Membranfluidpumpe verbundene zweite Auslasskanüle zum Ableiten des Fluids aus der zweiten Membranfluidpumpe,
eine zweite Arbeitspumpe,
welche über eine zweite Druckleitung mit der zweiten Membranfluidpumpe verbunden ist und eingerichtet ist, über die zweite Druckleitung die zweite Membranfluidpumpe anzutreiben, und
einen zweiten Einlass- und/oder zweiten Auslassflusssensor zum Erfassen eines zweiten Einlassflusses in der zweiten Einlasskanüle und/oder eines zweiten Auslassflusses in der zweiten Auslasskanüle,
wobei die Steuereinheit eingerichtet ist, die erste und/oder zweite Arbeitspumpe basierend auf dem erfassten ersten und/oder zweiten Einlass- und/oder Auslassfluss zu steuern.

13. Pumpensystem nach einem der vorhergehenden Ansprüche, **dadurch gekennzeichnet, dass** der erste und/oder zweite Einlass- und/oder Auslassflusssensor einen Ultraschallsensor umfasst.

14. Herzunterstützungssystem umfassend ein Pumpensystem nach einem der vorhergehenden Ansprüche zum Pumpen von Blut, **dadurch gekennzeichnet, dass** die erste und/oder zweite Einlasskanüle mit einer Herzkammer und/oder einem Vorhof eines Herzens und die erste und/oder zweite Auslasskanüle mit einem Blutgefäß fluidisch verbindbar ist.

15. Verfahren zum Betrieb eines Pumpensystems nach einem der Ansprüche 1 bis 13, **dadurch gekennzeichnet, dass** die erste und/oder zweite Arbeitspumpe basierend auf dem erfassten ersten und/oder zweiten Einlass- und/oder Auslassfluss gesteuert wird.
